(19) Europäisches Patentamt
European Patent Office
Office européen des brevets

(11) EP 0 835 094 B1

(12) **FASCICULE DE BREVET EUROPEEN**

(45) Date de publication et mention de la délivrance du brevet:
**18.06.2003 Bulletin 2003/25**

(21) Numéro de dépôt: **97919480.0**

(22) Date de dépôt: **03.04.1997**

(51) Int Cl.[7]: **A61K 7/42**

(86) Numéro de dépôt international:
**PCT/FR97/00606**

(87) Numéro de publication internationale:
**WO 97/037634 (16.10.1997 Gazette 1997/44)**

(54) **COMPOSITIONS COSMETIQUES FILTRANTES CONTENANT UN DIBENZOYLMETHANE, UN BETA'-DIPHENYLACRYLATE D'ALKYLE ET UNE SILICONE BENZOTRIAZOLE**

FILTRIERENDE KOSMETISCHE ZUSAMMENSETZUNGEN ENTHALTEND EIN DIBENZOYLMETHAN, EIN BETA-ALKYL-DIPHENYLACRYLAT UND EIN BENZOTRIAZOL-SILIKON-POLYMER

COSMETIC FILTER COMPOSITIONS CONTAINING DIBENZOYLMETHANE, ALKYL BETA'-DIPHENYLACRYLATE AND BENZOTRIAZOLE SILICONE

(84) Etats contractants désignés:
**AT BE CH DE DK ES FI FR GB GR IE IT LI LU MC NL PT SE**

(30) Priorité: **05.04.1996 FR 9604360**

(43) Date de publication de la demande:
**15.04.1998 Bulletin 1998/16**

(73) Titulaire: **L'OREAL**
**75008 Paris (FR)**

(72) Inventeur: **ASCIONE, Jean-Marc**
**Hoboken, NJ 07030 (US)**

(74) Mandataire: **Miszputen, Laurent**
**L'OREAL-DPI**
**6 rue Bertrand Sincholle**
**92585 Clichy Cédex (FR)**

(56) Documents cités:
**WO-A-91/11989**
**FR-A- 2 642 968**
**FR-A- 2 695 560**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 835 094 B1

Printed by Jouve, 75001 PARIS (FR)

## Description

La présente invention concerne de nouvelles compositions cosmétiques plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément, elle concerne de nouvelles compositions cosmétiques présentant un facteur de protection solaire amélioré et comprenant, dans un support cosmétiquement acceptable, au moins un dérivé de dibenzoylméthane, au moins un dérivé de β,β'-diphénylacrylate d'alkyle et au moins une silicone benzotriazole spécifique.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est-à-dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire (SPF) s'exprimant mathématiquement par le rapport du temps d'irradiation nécessaire pour atteindre le seuil érythématogène avec le filtre UV au temps nécessaire pour atteindre le seuil érythématogène sans filtre UV).

A la suite d'importantes recherches menées dans le domaine de la photoprotection évoqué ci-dessus, la Demanderesse a découvert, de façon inattendue et surprenante, qu'une combinaison de trois familles de composés filtrants particulières et déjà connues en soi dans l'état de l'art, permettait, du fait d'un effet de synergie, d'obtenir des compositions antisolaires présentant des facteurs de protection solaire nettement améliorés.

Cette découverte est à la base de la présente invention.

Ainsi, conformément à l'un des objets de la présente invention, il est maintenant proposé de nouvelles compositions cosmétiques, en particulier antisolaires, caractérisées par le fait qu'elles comprennent, dans un support cosmétiquement acceptable, i) au moins un dérivé de dibenzoylméthane à titre de premier filtre, ii) au moins un dérivé de β,β'-diphénylacrylate d'alkyle à titre de second filtre et iii) au moins une silicone benzotriazole répondant à l'une des formules suivantes :

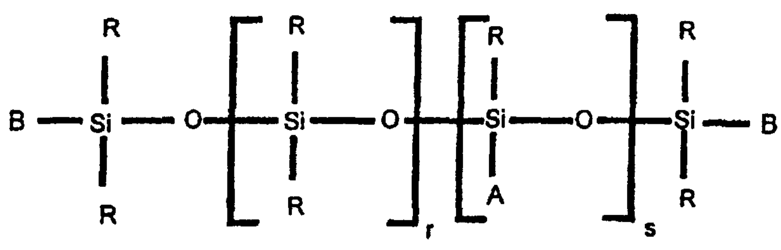

(1)

ou

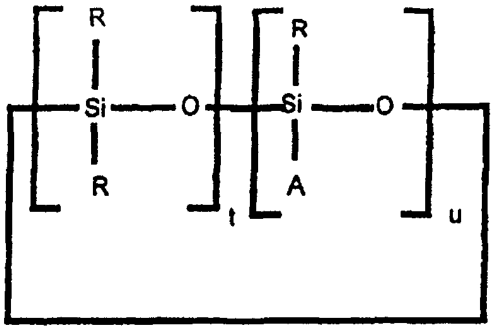

(2)

formules (1) et (2) dans lesquelles :

- R, identiques ou différents, sont choisis parmi les radicaux alkyles en $C_1$-$C_{10}$, phényle et trifluoro-3,3,3 propyle et triméthylsilyloxy, au moins 80% en nombre des radicaux R étant méthyle,
- B, identiques ou différents sont choisis parmi les radicaux R et le radical A,
- r est un nombre entier compris entre 0 et 50 inclusivement, et s est un nombre entier compris entre 0 et 20 inclusivement, et si s = 0, au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (3) suivante :

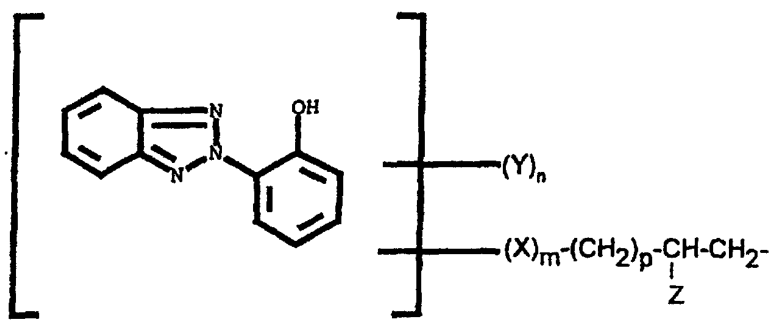

(3)

formule (3) dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement,

à titre de troisième filtre.

La présente invention a également pour objet un procédé de traitement cosmétique pour protéger la peau et/ou les cheveux contre le rayonnement UV, en particulier le rayonnement solaire, caractérisé en ce qu'il consiste à appliquer sur ces derniers une quantité efficace d'une composition cosmétique telle que définie ci-dessus.

D'autres caractéristiques, aspects et avantages de la présente invention apparaîtront à la lecture de la description détaillée qui va suivre.

Un premier filtre essentiel (appelé ci-après filtre A) des compositions selon l'invention est un dérivé de dibenzoylméthane. Les dérivés du dibenzoylméthane sont des produits déjà bien connus en soi comme agents filtrant les rayonnements UV-A et sont décrits notamment dans les documents FR-A- 2 326 405, FR-A- 2 440 933 et EP-A- 0 114 607, documents dont les enseignements sont, pour ce qui touche à la définition même de ces produits, totalement inclus à titre de références dans la présente description.

Parmi les dérivés du dibenzoylméthane plus particulièrement visés par la présente invention, on peut notamment citer, de manière non limitative :

- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- 4-tert-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane.

Parmi les dérivés du dibenzoylméthane mentionnés ci-dessus, on préfère tout particulièrement, selon la présente invention, mettre en oeuvre le 4-tert-butyl-4'-méthoxydibenzoylméthane, notamment celui proposé à la vente sous la dénomination commerciale de "PARSOL 1789" par la Société Givaudan, ce filtre répondant à la formule développée (I) suivante :

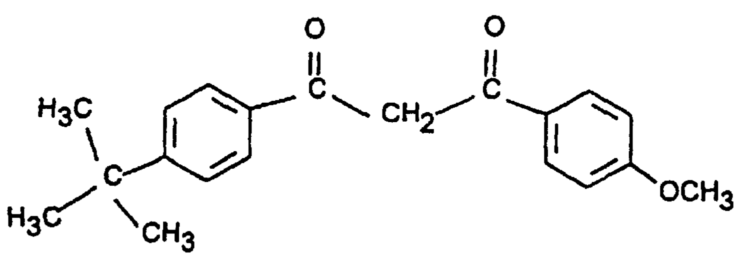

(I)

Un autre dérivé du dibenzoylméthane préféré selon la présente invention est le 4-isopropyldibenzoylméthane, filtre vendu sous la dénomination de "EUSOLEX 8020" par la Société Merck, et répondant à la formule développée (II) suivante :

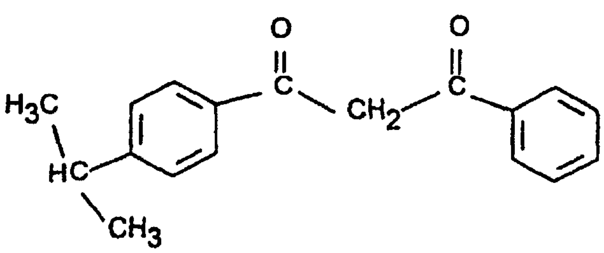

(II)

Le dérivé de dibenzoylméthane peut être présent dans les compositions conformes à l'invention à une teneur comprise entre 0,1 % et 8 %, de préférence entre 0,2 % et 5 % en poids, par rapport au poids total de la composition.

Un deuxième filtre essentiel (appelé ci-après filtre B) des compositions de l'invention est un dérivé de β,β'-diphénylacrylate d'alkyle. Parmi les dérivés de β,β'-diphénylacrylate d'alkyle utilisables selon la présente invention, on peut citer l'α-cyano-β,β' diphénylacrylate de 2-éthylhexyle ou encore l'α-cyano-β, β'-diphénylacrylate d'éthyle.

L'α-cyano-β,β' diphénylacrylate de 2-éthylhexyle, encore appelé octocrylène, est un filtre lipophile liquide déja

connu en soi pour son activité dans l'UV-B. Il s'agit d'un produit qui est disponible commercialement, et est vendu notamment sous la dénomination de "UVINUL N 539" par la Société BASF. Il répond à la formule (III) suivante :

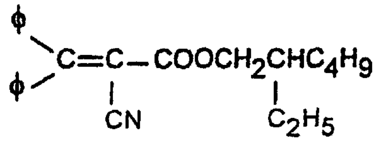

(III)

dans laquelle ϕ désigne un radical phényle.

L'α-cyano-β,β' diphénylacrylate d'éthyle, encore appelé étocrylène, est un filtre également connu en soi pour son activité dans l'UV-B. Il s'agit d'un produit qui est disponible commercialement, et est vendu notamment sous la dénomination de "UVINUL N 35" par la Société BASF. Il répond à la formule (IV) suivante :

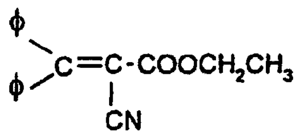

(IV)

dans laquelle ϕ désigne un radical phényle.

D'une manière plus générale, les β,β'-diphénylacrylates d'alkyle utilisables dans le cadre de la présente invention sont ceux déjà décrits dans la demande de brevet européen EP-A-0 514 491, dont l'enseignement est à cet égard totalement inclus dans la présente demande. On notera que le document EP-A-0 514 491 précité enseigne que les dérivés de β,β'-diphénylacrylates d'alkyle permettent de stabiliser photochimiquement les dérivés de dibenzoylméthane afin de garantir, pour cette association binaire, une protection constante au cours du temps, en particulier durant une exposition prolongée. Toutefois, ce document ne décrit ni ne suggère en en rien l'effet de synergie attaché à l'association ternaire conforme à l'invention au niveau des SPF intrinsèques initiaux.

De préférence, le dérivé de β,β-diphénylacrylate d'alkyle est présent dans les compositions selon l'invention à une teneur comprise entre 0,1 % et 20 % en poids, par rapport au poids total de la composition. De préférence encore, cette teneur est comprise entre 0,2 % et 15 % en poids, par rapport au poids total de la composition.

Un troisième filtre essentiel (appelé ci-après filtre C) des compositions conformes à la présente invention est une silicone benzotriazole spécifique. Les silicones benzotriazoles spécifiques utilisées dans le cadre de la présente invention sont sélectionnées au sein de la famille générale connue des silicones benzotriazoles, et sont celles qui répondent aux formules suivantes:

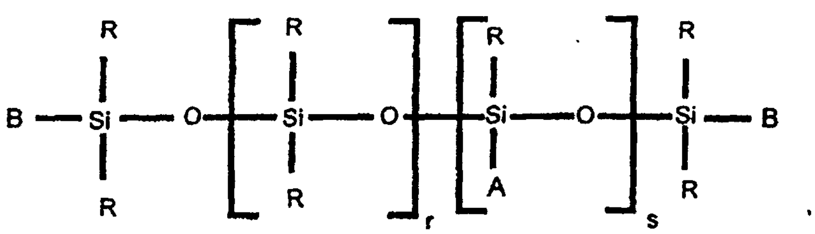

(1)

ou

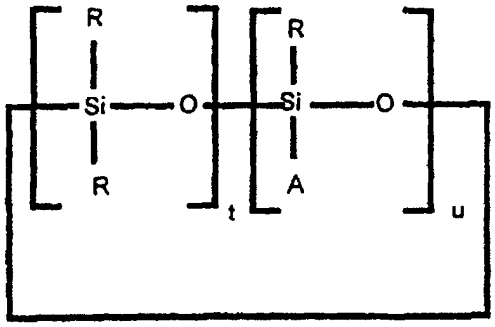

(2)

formules (1) et (2) dans lesquelles :

- R, identiques ou différents, sont choisis parmi les radicaux alkyles en $C_1$-$C_{10}$, phényle et trifluoro-3,3,3 propyle et triméthylsilyloxy, au moins 80% en nombre des radicaux R étant méthyle,
- B, identiques ou différents sont choisis parmi les radicaux R et le radical A,
- r est un nombre entier compris entre 0 et 50 inclusivement, et s est un nombre entier compris entre 0 et 20 inclusivement, et si s = 0, au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (3) suivante :

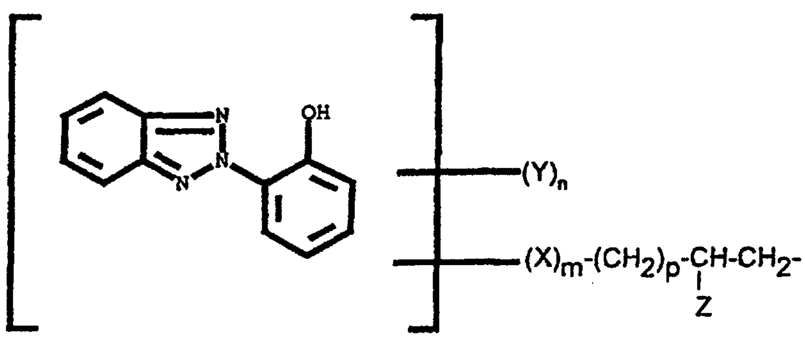

(3)

formule (3) dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement.

Comme cela ressort de la formule (3) donnée ci-dessus, l'accrochage du chaînon $-(X)_m-(CH_2)_p-CH(Z)-CH_2-$ sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazole :

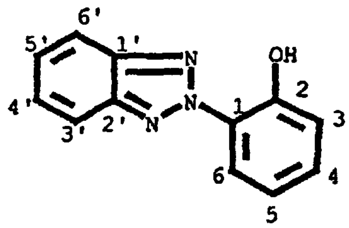

De préférence, cet accrochage se fait en position 3, 4, 5 (noyau aromatique portant la fonction hydroxy) ou 4' (noyau benzénique adjacent le cycle triazolé), et encore plus préférentiellement en position 3, 4 ou 5. Dans une forme préférée de réalisation de l'invention, l'accrochage se fait en position 3.

De même, l'accrochage du ou des motifs substituants Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage du motif Y se fait en position 5.

Dans les formules (1) et (2) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R préférés selon l'invention sont les radicaux méthyle, éthyle, propyle, n-butyle, n-octyle et éthyl-2 hexyle. Encore plus préférentiellement, les radicaux R sont tous des radicaux méthyle.

Parmi les composés de formules (1) ou (2) ci-dessus, on préfère mettre en oeuvre ceux répondant à la formule (1), c'est-à-dire des diorganosiloxanes à chaîne courte linéaire.

Parmi les composés de formules (1) ci-dessus, on préfère mettre en oeuvre ceux pour lesquels les radicaux B sont tous les deux des radicaux R.

Parmi les diorganosiloxanes linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :

- B est un radical R,
- R est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, ter.-butyle ou alcoxy en $C_1$-$C_4$,
- Z est hydrogène ou méthyle,
- m=0, ou [m=1 et X=O]
- p est égal à 1.

Une famille de composés convenant particulièrement à l'invention est celle définie par la formule générale (4) suivante :

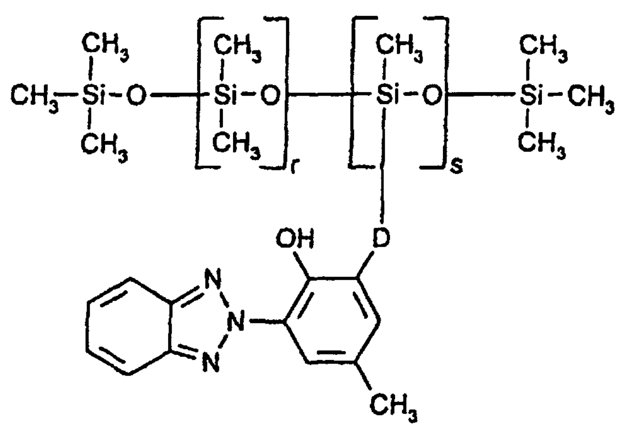

(4)

avec

$$0 \leq r \leq 10,$$

$$1 \leq s \leq 10,$$

et où D représente le radical divalent :

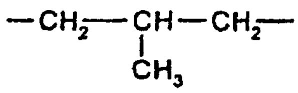

Dans une forme particulièrement préférée de réalisation de l'invention, la silicone benzotriazole est le composé (appelé composé (c) dans la suite du texte) répondant à la formule suivante :

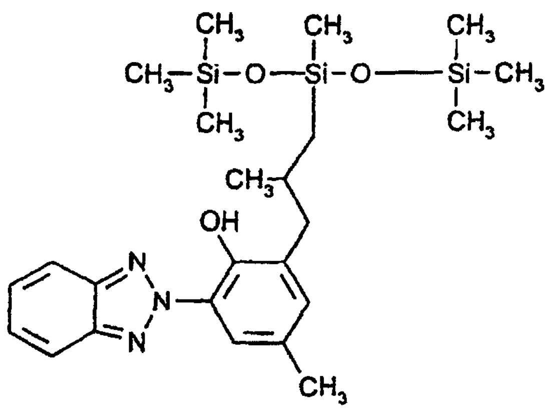

composé (c)

Pour préparer les filtres siliconés de formule (1) et (2), on peut procéder classiquement en mettant en oeuvre une réaction d'hydrosylilation (à savoir

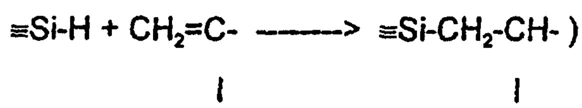

à partir de la silicone correspondante dans laquelle, par exemple, tous les radicaux A sont des atomes d'hydrogène. Cette silicone de départ est dénommée par la suite dérivé à SiH. Ces dérivés à SiH sont des produits bien connus dans l'industrie des silicones et sont généralement disponibles dans le commerce. Ils sont par exemple décrits dans les brevets américains US-A- 3 220 972, US-A- 3 697 473 et US-A- 4 340 709.

Ce dérivé à SiH peut être donc représenté soit par la formule (1bis) suivante :

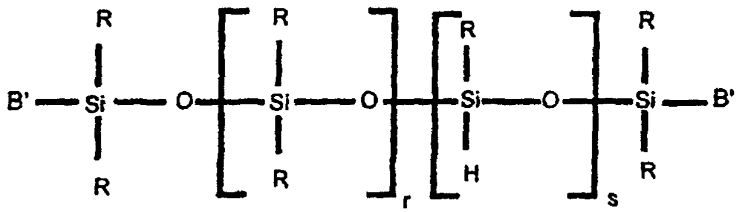

(1bis)

dans laquelle R, r et s ont la signification donnée ci-dessus pour la formule (1), et les radicaux B', identiques ou différents, sont choisis parmi les radicaux R et un atome d'hydrogène,
soit par la formule (2bis) suivante :

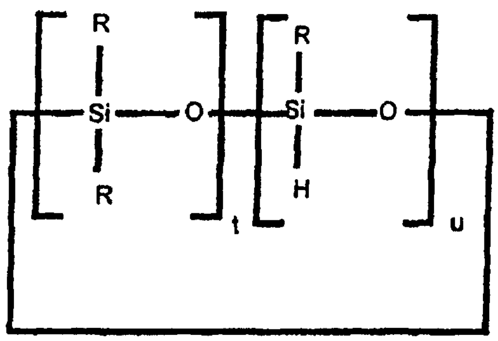

(2bis)

dans laquelle R, t et u ont la signification donnée ci-dessus pour la formule (2).

Sur ce dérivé à SiH de formules (1bis) ou (2bis), on effectue donc une réaction d'hydrosilylation classique, opérée en présence d'une quantité catalytiquement efficace d'un catalyseur au platine, sur un dérivé organique de benzotriazole de formule (3bis) suivante :

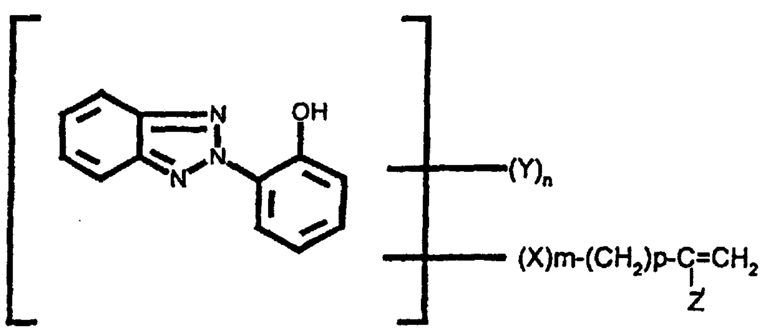

(3bis)

dans laquelle Y, X, Z, n, m et p ont la signification donnée ci-dessus pour la formule (3).

Des procédés convenant à la préparation des produits de formule (3bis) ci-dessus sont notamment décrits dans les brevets US- 4 316 033 et US- 4 328 346.

En outre, les détails des conditions opératoires à suivre pour conduire la réaction d'hydrosylilation entre les composés de formule (1bis) ou (2bis) ci-dessus avec le composé de formule (3bis) ci-dessus sont donnés dans la demande de brevet EP- 0 392 883, dont l'enseignement est, à cet égard, totalement inclus à titre de référence dans la présente description.

Dans les compositions de l'invention, on utilise généralement de 0,1 à 15 % en poids, de préférence de 0,2 à 10 % en poids, par rapport au poids total de la composition, d'une silicone benzotriazole telle que définie ci-dessus.

D'un point de vue pratique, les trois filtres A, B et C ci-dessus sont bien entendu de préférence tous trois présents dans la composition finale dans des proportions respectives choisies de manière telle que l'effet de synergie,

au niveau du facteur de protection solaire conféré par l'association résultante, soit optimal. La plage exacte des rapports pondéraux [filtre A/filtre B/filtre C] dans laquelle cet effet de synergie optimal est effectivement atteint peut varier légèrement selon la quantité totale de filtres A, B et C mise en oeuvre.

Dans une forme particulièrement préférée de réalisation de l'invention, le rapport pondéral [filtre A/filtre B/filtre C] est de 1/1/1.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les trois filtres mentionnés ci-avant. Ces filtres complémentaires peuvent être notamment choisis parmi les dérivés cinnamiques, les dérivés salicyliques, les dérivés du camphre, les dérivés de triazine, les dérivés de la benzophénone et les dérivés de l'acide p-aminobenzoïque.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A- 0 518 772 et EP-A- 0 518 773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones , les $\alpha$-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-$\alpha$-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose, l'hydroxypropylméthylcellulose ou encore l'hydroxyéthylcellulose.

Bien entendu, l'homme de l'art veillera à choisir le ou les éventuels composés complémentaires cités ci-dessus et/ou leurs quantités de manière telle que les propriétés avantageuses attachées intrinsèquement à l'association ternaire conforme à l'invention ne soient pas, ou substantiellement pas, altérées par la ou les adjonctions envisagées.

Les compositions concernées par l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

De préférence, cette composition se présente sous la forme d'une émulsion huile-dans-eau.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou

après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Des exemples concrets, mais nullement limitatifs, illustrant l'invention, vont maintenant être donnés.

**<u>EXEMPLE 1 :</u>**

On a préparé diverses formulations antisolaires se présentant sous la forme d'une émulsion de type huile-dans-eau et contenant (les quantités sont exprimées en % de poids par rapport au poids total de la composition) :

- 4-(tert.butyl) 4'-méthoxy dibenzoylméthane x %
- α-cyano-β,β'-diphénylacrylate de 2-éthylhexyle y %
- silicone benzotriazole (composé (c) tel que défini ci-avant dans la description) z %
- mélange d'alcool cétyl stéarylique et d'alcool cétyl stéarylique oxyéthyléné à 33 moles d' OE (80/20) vendu sous la dénomination « Sinnovax AO » par Henkel 7 %
- monostéarate de glycérol vendu sous la dénomination commerciale « Géléol Copeaux » par Gattefossé 2 %
- alcool cétylique vendu sous la dénomination « Lorol C 16 » par Henkel 1,5%
- 2,2,4,4,6,6,8-heptaméthylnonane vendu sous la dénomination commerciale « Isohexadécane » par Bayer 15 %
- polydiméthylsiloxane vendu sous la dénomination « Silbione 70 047 V 300 » par Rhône-Poulenc 1,5%
- glycérine 20%
- conservateurs qs
- eau qsp 100 %

On a ainsi réalisé quatre émulsions : trois émulsions comparatives A, B, et C, chacune comprenant respectivement le 4-(tert.butyl) 4'-méthoxy dibenzoylméthane seul, l'α-cyano-β,β'-diphénylacrylate de 2-éthylhexyle seul et la silicone benzotriazole (c) seule, et une émulsion D conforme à l'invention, comprenant du (4-(tert.butyl) 4'-méthoxy dibenzoylméthane, de l'α-cyano-β,β'-diphénylacrylate de 2-éthylhexyle et la silicone benzotriazole (c)). Les quantités respectives en ces trois filtres dans les quatre émulsions A, B, C et D sont rassemblées dans le tableau (I) ci-dessous :

Tableau (I) :

| Constituant | Formule A (comparative) | Formule B (comparative) | Formule C (comparative) | Formule D (invention) |
|---|---|---|---|---|
| x (%) | 6 | 0 | 0 | 2 |
| y (%) | 0 | 6 | 0 | 2 |
| z (%) | 0 | 0 | 6 | 2 |

Pour chacune de ces formulations, on a ensuite déterminé le facteur de protection solaire (SPF) qui lui était attaché. Celui-ci a été déterminé en utilisant la méthode *in vitro* décrite par B.L. DIFFEY et al. dans J. Soc. Cosmet. Chem. 40-127-133 (1989) ; cette méthode consiste à déterminer les facteurs de protection monochromatiques tous les 5 nm dans une gamme de longueurs d'onde de 290 à 400 nm et à calculer à partir de ceux-ci le facteur de protection solaire selon une équation mathématique donnée.

Les résultats (valeur moyenne correspondant à trois essais) sont regroupés dans le tableau (II) ci-dessous :

Tableau (II) :

| Formule | A (comparative) | B (comparative) | C (comparative) | D (invention) |
|---|---|---|---|---|
| SPF | 3,6 | 5,0 | 4,8 | 8,5 |

Ces résultats montrent clairement l'effet de synergie obtenu avec la composition D conforme à l'invention. Le SPF de la composition D comprenant le mélange synergique de trois filtres (4-(tert.butyl) 4'-méthoxy dibenzoylméthane, α-cyano-β,β'-diphénylacrylate de 2-éthylhexyle et silicone benzotriazole (c)) selon l'invention est significativement supérieur à celui de chacune des formules A, B et C qui comprennent respectivement le 4-(tert.butyl) 4'-méthoxy dibenzoylméthane seul (formule A), l'α-cyano-β,β'-diphénylacrylate de 2-éthylhexyle seul (formule B) et la silicone benzotriazole (c) seule (formule C), à des teneurs globales identiques.

**EXEMPLE 2 :**

On a également préparé les compositions comparatives E, F, G, H, J et K de support commun identique aux émulsions de l'exemple 1 et dont les proportions respectives en 4-(tert.butyl) 4'-méthoxy dibenzoylméthane, en α-cyano-β,β'-diphénylacrylate de 2-éthylhexyle et en silicone benzotriazole (c) sont rassemblées dans le tableau (III) suivant :

Tableau (III) :

| Filtre | Formule E | Formule F | Formule G | Formule H | Formule J | Formule K |
|---|---|---|---|---|---|---|
| x (%) | 2 | 0 | 0 | 0 | 2 | 2 |
| y (%) | 0 | 2 | 0 | 2 | 0 | 2 |
| z (%) | 0 | 0 | 2 | 2 | 2 | 0 |

Pour chacune de ces formulations, on a ensuite déterminé le facteur de protection solaire selon le même protocole que celui utilisé dans l'exemple 1.

Les résultats (valeur moyenne correspondant à trois essais) sont regroupés dans le tableau (IV) ci-dessous :

Tableau (IV) :

| Formule | E | F | G | H | J | K |
|---|---|---|---|---|---|---|
| SPF | 3,2 | 2,2 | 2,7 | 3,3 | 4,1 | 4,2 |

Ainsi, si à chaque SPF obtenu ci-dessus pour l'une des formules E, F ou G renfermant un seul des filtres x, y ou z, on additionne le SPF obtenu pour la composition H, J ou K comprenant les deux filtres complémentaires, on obtient des SPF nettement moins élevés que le SPF de la composition D selon l'invention de l'exemple 1 et qui comprend l'association synergique des trois filtres selon l'invention dans la même proportion globale de filtres de 6 %. Le tableau (V) ci-dessous résume ces valeurs :

Tableau (V) :

| SPF (formule E) + SPF (formule H) | SPF (formule F) + SPF (formule J) | SPF (formule G) + SPF (formule K) | SPF (**formule D**) (invention) |
|---|---|---|---|
| 6,5 | 6,3 | 6,9 | **8,5** |

**Revendications**

**1.** Composition cosmétique, en particulier antisolaire, **caractérisée par le fait qu'**elle comprend, dans un support

cosmétiquement acceptable, i) au moins un dérivé de dibenzoylméthane à titre de premier filtre, ii) au moins un dérivé de β,β'-diphénylacrylate d'alkyle à titre de second filtre et iii) au moins une silicone benzotriazole répondant à l'une des formules suivantes :

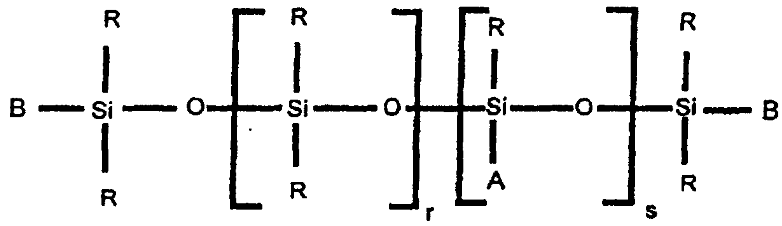

(1)

ou

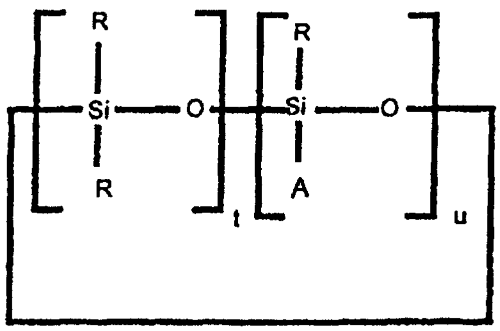

(2)

formules (1) et (2) dans lesquelles :

- R, identiques ou différents, sont choisis parmi les radicaux alkyles en $C_1$-$C_{10}$, phényle et trifluoro-3,3,3 propyle et triméthylsilyloxy, au moins 80% en nombre des radicaux R étant méthyle,
- B, identiques ou différents sont choisis parmi les radicaux R et le radical A,
- r est un nombre entier compris entre 0 et 50 inclusivement, et s est un nombre entier compris entre 0 et 20 inclusivement, et si s = 0, au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (3) suivante :

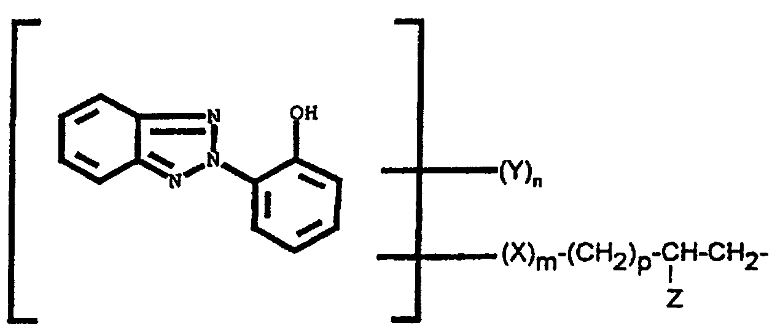

(3)

formule (3) dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement,

à titre de troisième filtre.

**2.** Composition selon la revendication 1, **caractérisée par le fait que** le dérivé de dibenzoylméthane est choisi parmi :

- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- 4-tert-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane.

**3.** Composition selon la revendication 2, **caractérisée par le fait que** le dérivé de dibenzoylméthane est le 4-tert-butyl-4'-méthoxydibenzoylméthane.

**4.** Composition selon la revendication 2, **caractérisée par le fait que** le dérivé de dibenzoylméthane est le 4-isopropyldibenzoylméthane.

**5.** Composition selon l'une quelconque des revendications précédentes, **caractérisée par le fait que** le dérivé de dibenzoylméthane est présent dans la composition à une teneur comprise entre 0,1 % et 8 % en poids, par rapport au poids total de la composition.

**6.** Composition selon la revendication 5, **caractérisée par le fait que** ladite teneur est comprise entre 0,2 % et 5 % en poids, par rapport au poids total de la composition.

**7.** Composition selon l'une quelconque des revendications précédentes, **caractérisée par le fait que** le dérivé de β,β-diphénylacrylate d'alkyle est choisi parmi l'α-cyano-β,β' diphénylacrylate de 2-éthylhexyle et l'α-cyano-β, β'-diphénylacrylate d'éthyle.

**8.** Composition selon la revendication 7, **caractérisée par le fait que** le dérivé de β,β-diphénylacrylate d'alkyle est l'α-cyano-β,β' diphénylacrylate de 2-éthylhexyle.

**9.** Composition selon l'une quelconque des revendications précédentes, **caractérisée par le fait que** le dérivé de β,β-diphénylacrylate d'alkyle est présent dans la composition à une teneur comprise entre 0,1 % et 20 % en poids, par rapport au poids total de la composition.

**10.** Composition selon la revendication 9, **caractérisée par le fait que** ladite teneur est comprise entre 0,2 % et 15 % en poids, par rapport au poids total de la composition.

**11.** Composition selon l'une quelconque des revendications précédentes, **caractérisée par le fait que** la silicone benzotriazole est choisie parmi les composés de formule générale (1) pour lesquels les radicaux B sont choisis

parmi les radicaux R.

**12.** Composition selon la revendication 11, **caractérisée par le fait que** la silicone benzotriazole est choisie au sein de celles présentant au moins l'une des caractéristiques suivantes :

- R est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, ter.-butyle ou alcoxy en $C_1$-$C_4$,
- Z est hydrogène ou méthyle,
- m=0, ou [m=1 et X=O]
- p est égal à 1.

**13.** Composition selon la revendication 12, **caractérisée par le fait que** ladite silicone benzotriazole présente l'ensemble desdites caractéristiques.

**14.** Composition selon la revendication 13, **caractérisée par le fait que** la silicone benzotriazole est choisie parmi les composés de formule générale (4) :

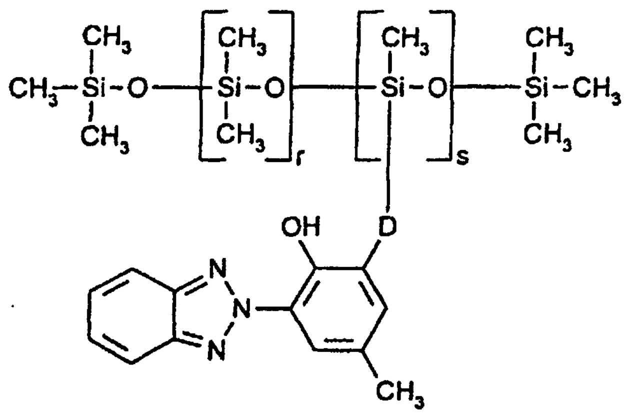

**(4)**

avec

$$0 \leq r \leq 10,$$

$$1 \leq s \leq 10,$$

et où D représente le radical divalent :

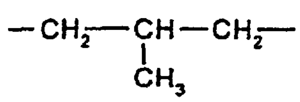

**15.** Composition selon la revendication 14, **caractérisée par le fait que** la silicone benzotriazole répond à la formule suivante :

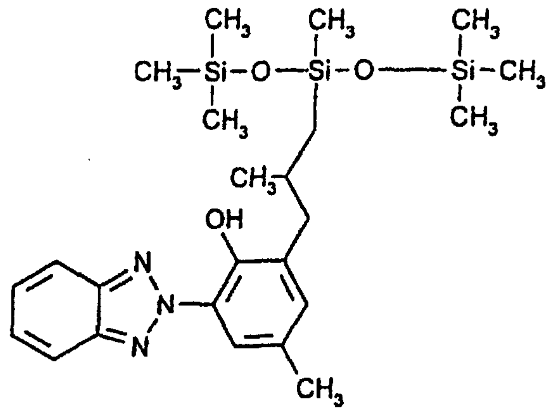

**16.** Composition selon l'une quelconque des revendications précédentes. **caractérisée par le fait que** ladite silicone benzotriazole est présente dans la composition à une teneur comprise entre 0,1 à 15 % en poids, par rapport au poids total de la composition.

**17.** Composition selon la revendication 16, **caractérisée par le fait que** ladite teneur est comprise entre 0,2 à 10 % en poids, par rapport au poids total de la composition.

**18.** Procédé de traitement cosmétique pour protéger la peau et/ou les cheveux contre le rayonnement UV, en particulier le rayonnement solaire, **caractérisé en ce qu'**il consiste à appliquer sur ces derniers une quantité efficace d'une composition cosmétique telle que définie à l'une quelconque des revendications 1 à 17.

**Claims**

**1.** Cosmetic composition, in particular an antisun composition, **characterized in that** it comprises, in a cosmetically acceptable vehicle, i) at least one dibenzoylmethane derivative as first screening agent, ii) at least one alkyl β,β'-diphenylacrylate derivative as second screening agent, and iii) at least one benzotriazole silicone corresponding to one of the following formulae:

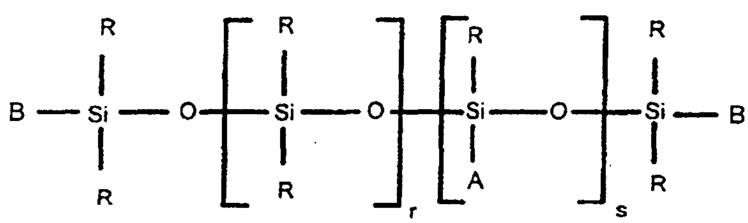

(1)

or

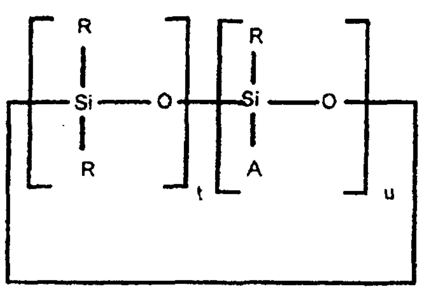

(2)

in which formulae (1) and (2):

- R, which may be identical or different, are chosen from $C_1$-$C_{10}$ alkyl, phenyl, 3,3,3-trifluoropropyl and trimethylsilyloxy radicals, at least 80%, on a number basis, of the radicals R being methyl,
- B, which may be identical or different, are chosen from the radicals R and the radical A,
- r is an integer between 0 and 50 inclusive and s is an integer between 0 and 20 inclusive, and if s = 0, at least one of the two symbols B denotes A,
- u is an integer between 1 and 6 inclusive and t is an integer between 0 and 10 inclusive, it being understood that t + u is equal to or greater than 3,
- and the symbol A denotes a monovalent radical linked directly to a silicon atom, and which corresponds to the formula (3) below:

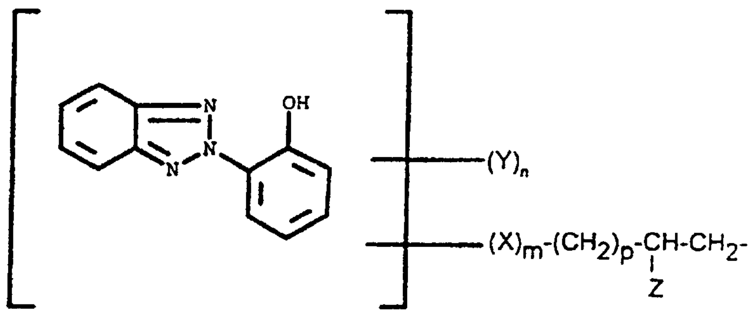

(3)

in which formula (3):

- Y, which may be identical or different, are chosen from $C_1$-$C_8$ alkyl radicals, halogens and $C_1$-$C_4$ alkoxy radicals, it being understood that, in this latter case, two adjacent Y groups on the same aromatic ring may together form an alkylidenedioxy group in which the alkylidene group contains 1 or 2 carbon atoms,
- X represents O or NH,
- Z represents hydrogen or a $C_1$-$C_4$ alkyl radical,
- n is an integer between 0 and 3 inclusive,
- m is 0 or 1,
- p represents an integer between 1 and 10 inclusive,

as third screening agent.

**2.** Composition according to Claim 1, **characterized in that** the dibenzoylmethane derivative is chosen from:

- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2,5-dimethyldibenzoylmethane,
- 4,4'-diisopropyldibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane.

**3.** Composition according to Claim 2, **characterized in that** the dibenzoylmethane derivative is 4-tert-butyl-4'-methoxydibenzoylmethane.

**4.** Composition according to Claim 2, **characterized in that** the dibenzoylmethane derivative is 4-isopropyldibenzoylmethane.

5. Composition according to any one of the preceding claims, **characterized in that** the dibenzoylmethane derivative is present in the composition at a content of between 0.1% and 8% by weight relative to the total weight of the composition.

6. Composition according to Claim 5, **characterized in that** the said content is between 0.2% and 5% by weight relative to the total weight of the composition.

7. Composition according to any one of the preceding claims, **characterized in that** the alkyl β,β'-diphenylacrylate derivative is chosen from 2-ethylhexyl α-cyano-β,β'-diphenylacrylate and ethyl α-cyano-β,β'-diphenylacrylate.

8. Composition according to Claim 7, **characterized in that** the alkyl β,β'-diphenylacrylate derivative is 2-ethylhexyl α-cyano-β,β'-diphenylacrylate.

9. Composition according to any one of the preceding claims, **characterized in that** the alkyl β,β'-diphenylacrylate derivative is present in the composition at a content of between 0.1% and 20% by weight relative to the total weight of the composition.

10. Composition according to Claim 9, **characterized in that** the said content is between 0.2% and 15% by weight relative to the total weight of the composition.

11. Composition according to any one of the preceding claims, **characterized in that** the benzotriazole silicone is chosen from the compounds of general formula (1) for which the radicals B are chosen from the radicals R.

12. Composition according to Claim 11, **characterized in that** the benzotriazole silicone is chosen from those having at least one of the following characteristics:

- R is alkyl and even more preferably is methyl,
- r is between 0 and 15 inclusive; s is between 1 and 10 inclusive,
- n is non-zero and preferably equal to 1, and Y is then chosen from methyl, tert-butyl and $C_1$-$C_4$ alkoxy,
- Z is hydrogen or methyl,
- m=0 or [m=1 and X=O]
- p is equal to 1.

13. Composition according to Claim 12, **characterized in that** the said benzotriazole silicone has all of the said characteristics.

14. Composition according to Claim 13, **characterized in that** the benzotriazole silicone is chosen from compounds of general formula (4) :

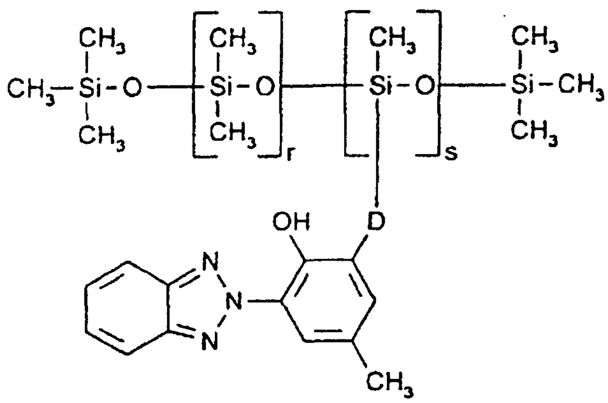

(4)

with

$$0 \leq r \leq 10,$$

$$1 \leq s \leq 10,$$

and where D represents the divalent radical:

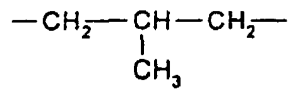

**15.** Composition according to Claim 14, **characterized in that** the benzotriazole silicone corresponds to the following formula:

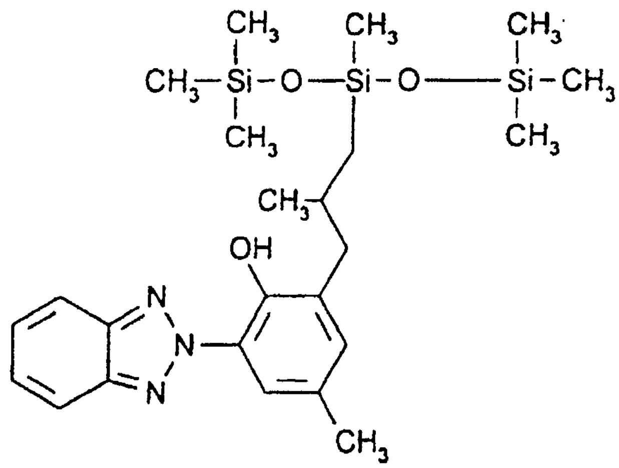

**16.** Composition according to any one of the preceding claims, **characterized in that** the said benzotriazole silicone is present in the composition at a content of between 0.1 and 15% by weight relative to the total weight of the composition.

**17.** Composition according to Claim 16, **characterized in that** the said content is between 0.2 and 10% by weight relative to the total weight of the composition.

**18.** Cosmetic treatment process for protecting the skin and/or the hair against UV radiation, in particular solar radiation, **characterized in that** it consists in applying thereto an effective amount of a cosmetic composition as defined in any one of Claims 1 to 17.

**Patentansprüche**

**1.** Kosmetische Zusammensetzung und insbesondere Sonnenschutzmittel, **dadurch gekennzeichnet, dass** sie in einem kosmetisch akzeptablen Träger (i) mindestens ein Dibenzoylmethanderivat als erstes Filter, (ii) mindestens ein Alkyl-β,β'-diphenylacrylatderivat als zweites Filter und (iii) als drittes Filter mindestens ein Benzotriazolsilicon enthält, das einer der folgenden Formeln entspricht:

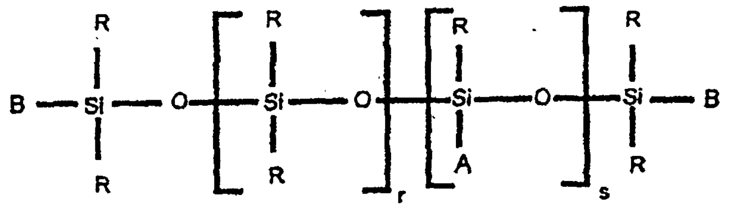

(1)

oder

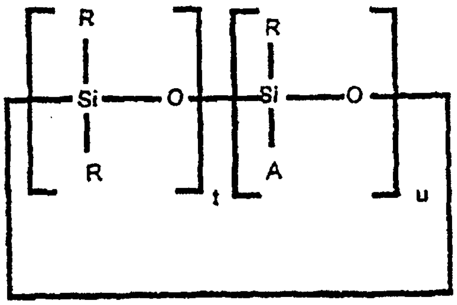

(2),

wobei in den Formeln (1) und (2):

- die Gruppen R, die identisch oder voneinander verschieden sind, unter den $C_{1-10}$-Alkylgruppen, Phenyl, 3,3,3-Trifluorpropyl und Trimethylsilyloxy ausgewählt sind, wobei mindestens 80 % der Anzahl der Gruppen R Methyl bedeutet,
- die Gruppen B, die identisch oder voneinander verschieden sind, unter den Gruppen R und der Gruppe A ausgewählt sind,
- r 0 oder eine ganze Zahl im Bereich von 1 bis 50 und s 0 oder eine ganze Zahl im Bereich von 1 bis 20 bedeutet, und mindestens eine der beiden Gruppen B A bedeutet, wenn s 0 ist,
- u eine ganze Zahl im Bereich von 1 bis 6 und t 0 oder eine ganze Zahl im Bereich von 1 bis 10 ist, mit der Maßgabe, dass t + u 3 bedeutet oder darüber liegt, und
- die Gruppe A eine einwertige Gruppe ist, die direkt an ein Siliciumatom gebunden ist und der folgenden Formel (3) entspricht:

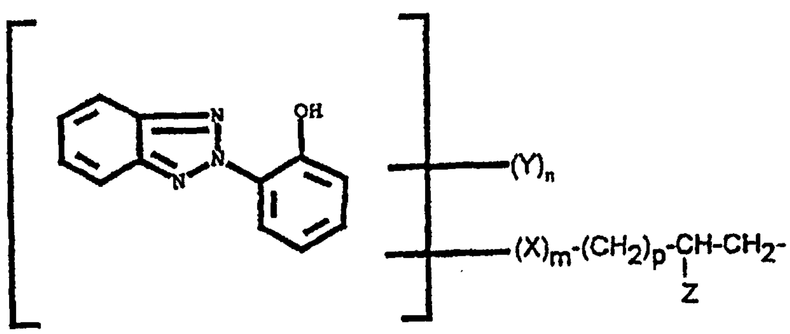

(3),

wobei in der Formel (3):

- die Gruppen Y, die identisch oder voneinander verschieden sind, unter den $C_{1-8}$-Alkylgruppen, Halogenen und $C_{1-4}$-Alkoxygruppen ausgewählt sind, wobei im letzten Fall zwei an dem gleichen aromatischen Ring aneinander angrenzende Gruppen Y auch gemeinsam eine Alkylidendioxygruppe bilden können,

worin die Alkylidengruppe 1 bis 2 Kohlenstoffatome aufweist,

- X O oder NH bedeutet,
- Z Wasserstoff oder eine $C_{1-4}$-Alkylgruppe bedeutet,
- n 0 oder eine ganze Zahl im Bereich von 1 bis 3 ist,
- m 0 oder 1 ist, und
- p eine ganze Zahl im Bereich von 1 bis 10 bedeutet.

**2.** Zusammensetzung nach Anspruch 1, **dadurch gekennzeichnet, dass** das Dibenzoylmethanderivat ausgewählt ist unter:

- 2-Methyldibenzoylmethan,
- 4-Methyldibenzoylmethan,
- 4-Isopropyldibenzoylmethan,
- 4-*t*-Butyldibenzoylmethan,
- 2,4-Dimethyldibenzoylmethan,
- 2,5-Dimethyldibenzoylmethan,
- 4,4'-Diisopropyldibenzoylmethan,
- 4-*t*-Butyl-4'-methoxy-dibenzoylmethan,
- 2-Methyl-5-isopropyl-4'-methoxydibenzoylmethan,
- 2-Methyl-5-*t*-butyl-4'-methoxydibenzoylmethan,
- 2,4-Dimethyl-4'-methoxydibenzoylmethan, und
- 2,6-Dimethyl-4-*t*-butyl-4'-methoxydibenzoylmethan.

**3.** Zusammensetzung nach Anspruch 2, **dadurch gekennzeichnet, dass** das Dibenzoylmethanderivat das 4-*t*-Butyl. 4'-methoxydibenzoylmethan ist.

**4.** Zusammensetzung nach Anspruch 2, **dadurch gekennzeichnet, dass** das Dibenzoylmethanderivat das 4-Isopropyldibenzoylmethan ist.

**5.** Zusammensetzung nach einem der vorhergehenden Ansprüche, **dadurch gekennzeichnet, dass** das Dibenzoylmethanderivat in der Zusammensetzung in einer Menge von 0,1 bis 8 Gew.-%, bezogen auf das Gesamtgewicht der Zusammensetzung, vorliegt.

**6.** Zusammensetzung nach Anspruch 5, **dadurch gekennzeichnet, dass** der Mengenanteil im Bereich von 0,2 bis 5 Gew.-%, bezogen auf das Gesamtgewicht der Zusammensetzung, liegt.

**7.** Zusammensetzung nach einem der vorhergehenden Ansprüche, **dadurch gekennzeichnet, dass** das Alkyl-$\beta,\beta'$-diphenylacrylatderivat unter 2-Ethylhexyl-$\alpha$-cyano-$\beta,\beta'$-diphenylacrylat und Ethyl-$\alpha$-cyano-$\beta,\beta'$-diphenylacrylat ausgewählt ist.

**8.** Zusammensetzung nach Anspruch 7, **dadurch gekennzeichnet, dass** das Alkyl-$\beta,\beta'$-diphenylacrylatderivat das 2-Ethylhexyl-$\alpha$-cyano-$\beta,\beta'$-diphenylacrylat ist.

**9.** Zusammensetzung nach einem der vorhergehenden Ansprüche, **dadurch gekennzeichnet, dass** das Alkyl-$\beta,\beta'$-diphenylacrylatderivat in der Zusammensetzung in einer Menge von 0,1 bis 20 Gew.-%, bezogen auf das Gesamtgewicht der Zusammensetzung, vorliegt.

**10.** Zusammensetzung nach Anspruch 9, **dadurch gekennzeichnet, dass** der Mengenanteil im Bereich von 0,2 bis 15 Gew.-%, bezogen auf das Gesamtgewicht der Zusammensetzung, liegt.

**11.** Zusammensetzung nach einem der vorhergehenden Ansprüche, **dadurch gekennzeichnet, dass** das Benzotriazolsilicon unter den Verbindungen der allgemeinen Formel (1) ausgewählt ist, worin die Gruppen B unter den Gruppen R ausgewählt sind.

**12.** Zusammensetzung nach Anspruch 11, **dadurch gekennzeichnet, dass** das Benzotriazolsilicon unter den Verbindungen ausgewählt ist, die mindestens eine der folgenden Eigenschaften aufweisen:

- R bedeutet Alkyl und noch bevorzugter Methyl,
- r liegt im Bereich von 0 bis 15; s liegt im Bereich von 1 bis 10,
- n ist nicht Null und vorzugsweise 1, und Y ist unter Methyl, *t*-Butyl oder $C_{1-4}$-Alkoxy ausgewählt,
- Z bedeutet Wasserstoff oder Methyl,
- m = 0, oder [m = 1 und X = O], und
- p bedeutet 1.

**13.** Zusammensetzung nach Anspruch 12, **dadurch gekennzeichnet, dass** das Benzotriazolsilicon alle diese Eigenschaften aufweist.

**14.** Zusammensetzung nach Anspruch 13, **dadurch gekennzeichnet, dass** das Benzotriazolsilicon unter den Verbindungen der allgemeinen Formel (4) ausgewählt ist:

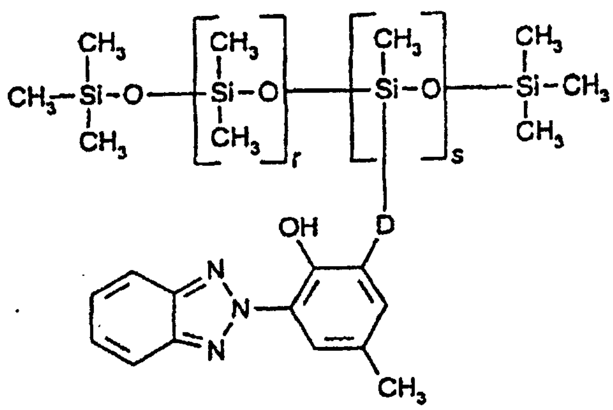

(4)

worin bedeuten:

$$0 \leq r \leq 10,$$

$$1 \leq s \leq 10,$$

und D die zweiwertige Gruppe:

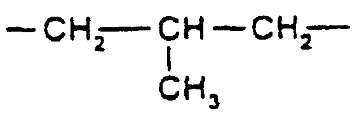

**15.** Zusammensetzung nach Anspruch 14, **dadurch gekennzeichnet, dass** das Benzotriazolsilicon der folgenden Formel entspricht:

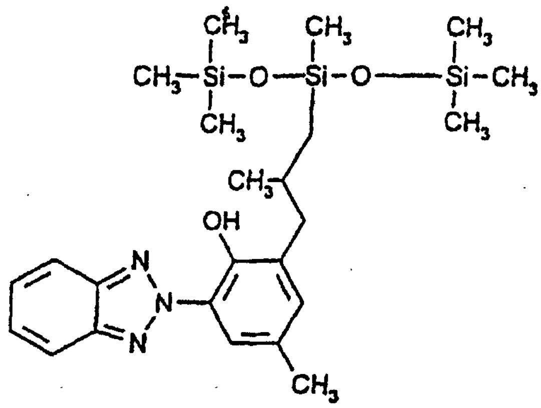

**16.** Zusammensetzung nach einem der vorhergehenden Ansprüche, **dadurch gekennzeichnet, dass** das das Benzotriazolsilicon in der Zusammensetzung in einer Menge von 0,1 bis 15 Gew.-%, bezogen auf das Gesamtgewicht der Zusammensetzung, vorliegt.

**17.** Zusammensetzung nach Anspruch 16, **dadurch gekennzeichnet, dass** der Mengenanteil im Bereich von 0,2 bis 10 Gew.-%, bezogen auf das Gesamtgewicht der Zusammensetzung, liegt.

**18.** Verfahren zum Schutz der Haut und/oder der Haare gegen UV-Strahlung und insbesondere Sonnenlicht, **dadurch gekennzeichnet, dass** es darin besteht, auf diese eine wirksame Menge einer kosmetischen Zusammensetzung nach einem der Ansprüche 1 bis 17 aufzutragen.